# EUROPEAN PATENT APPLICATION

(11) **EP 4 706 555 A1**
(43) Date of publication of application: **11.03.2026**
(21) Application number: 24797351.4
(22) Date of filing: 19.04.2024
(51) Int. Cl.: A61B 17/12, A61M 25/10, A61B 17/00

(54) **BALLOON CATHETER FOR BRONCHIAL HEMOSTASIS**

(30) Priority: 24.04.2023 KR 20230053266
(71) Applicant: CHUNGNAM NATIONAL UNIVERSITY HOSPITAL, Daejeon 35015 (KR)
(72) Inventor: PARK, Dongil, Daejeon 34933 (KR); CHUNG, Chaeuk, Daejeon 35248 (KR)
(74) Representative: Gille Hrabal Partnerschaftsgesellschaft mbB Patentanwälte
(86) International application number: PCT/KR2024/005285
(87) International publication number: WO 2024/225695

(57) **Abstract**

The present invention relates to a hemostatic balloon catheter for bronchoscopy that is prophylactically and stably secured in a bronchus to prepare for the possibility of massive bleeding during a bronchoscopic procedure, and is capable of achieving immediate hemostasis at a precise location upon the occurrence of bleeding, more specifically relates to a bronchial hemostatic balloon catheter, characterized in that a securing balloon is provided at a distal end to be inserted into the body, and one or more hemostatic balloons are provided at a predetermined distance from the securing balloon.

## Description

### Technical Field

The present invention relates to a hemostatic balloon catheter for bronchoscopy that is prophylactically and stably secured in a bronchus to prepare for the possibility of massive bleeding during a bronchoscopic procedure, and is capable of achieving immediate hemostasis at a precise location upon the occurrence of bleeding.

### Background Art

Bronchoscopy is a diagnostic method for respiratory diseases, which is a relatively safe examination with a moderate degree of invasiveness. According to bronchoscopy, various bronchial and pulmonary diseases can be diagnosed by directly observing, through the use of a bronchoscope, whether abnormalities exist in the mucosa of the pharynx, vocal cords, trachea, and bronchi. During the examination, procedures such as bronchial lavage, alveolar lavage, bronchial mucosal brushing, bronchial mucosal biopsy, lung tissue biopsy, and lymph node fine needle aspiration may be performed. Such bronchoscopy is not a commonly performed examination like gastroscopy, but it is a very important and effective examination for certain respiratory diseases. Through this, not only can the condition of the pharynx, vocal cords, trachea, bronchi, and alveoli be visually observed, but also important information for identifying the cause of disease can be obtained by approaching the affected area of the lung to collect secretions or tissue specimens.

There are cases where massive bleeding occurs from a lesion while a specimen is being collected during a bronchoscopic procedure. If blood flows into the central airway at this time, it may cause risks such as respiratory failure and oxygen supply impairment due to extensive airway obstruction. However, since most pulmonary lesions are located in the periphery, direct visualization is difficult, and there is no means to achieve hemostasis by direct compression of the lesion itself. Accordingly, when the operator discovers massive bleeding (or the possibility thereof), the operator inserts a hemostatic balloon catheter through the working channel of the bronchoscope (after stopping its withdrawal), inflates the balloon at the entrance of the bronchus where the bleeding lesion is located, and thereby blocks the bronchus connected to the lesion to prevent blood from flowing out.

However, when massive bleeding occurs from a lesion, the endoscopic view becomes obscured within a few seconds, making immediate response difficult. In addition, since the hemostatic balloon catheter is inserted through the working channel of the endoscope, once the balloon catheter is secured in place, the endoscope also becomes substantially immobilized. Therefore, until hemostasis is completed, not only are procedures such as internal observation and suction difficult, but the endoscope must also remain inserted in the patient until the bleeding is completely stopped, thereby causing difficulties for both the operator and the patient.

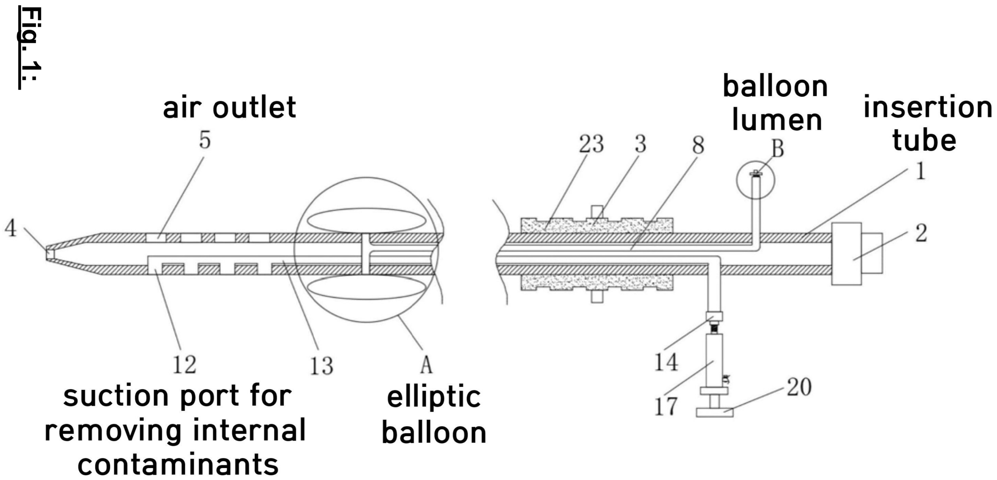

The Chinese Utility Model CN 213667381 (Title of the Invention: Tracheal intubation device suitable for bronchoscope operation hemoptysis rescue and difficult airway) relates to a balloon catheter wherein a single balloon is provided at the distal side of the catheter so that, when bleeding occurs in the trachea, the balloon is inflated at the bleeding site to stop the bleeding by compressing the bleeding site, while allowing air to pass through the tube catheter to enable breathing, and further comprising a suction port for removing internal contaminants such as blood (see Fig. 1). However, this is intended to achieve hemostasis by compressing the already identified bleeding site itself, and therefore cannot be used for hemostasis of bleeding occurring in the periphery of the lung, and it cannot be used simultaneously with a bronchoscope.

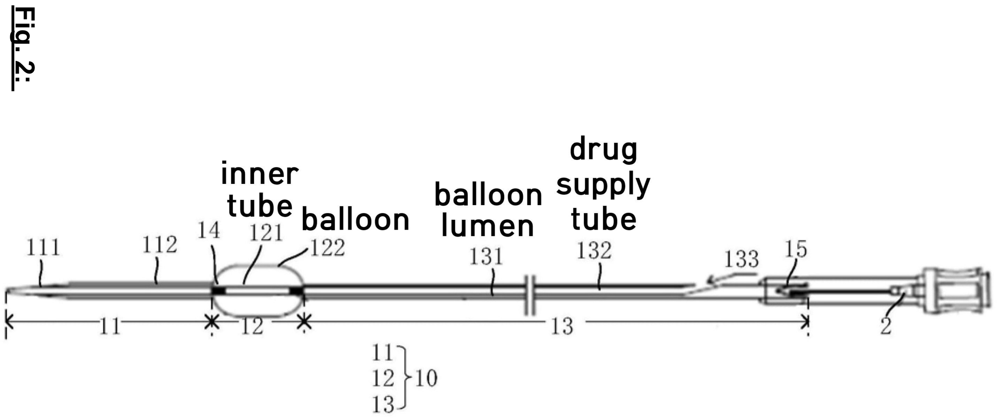

The Chinese Utility Model CN 215275335 (Title of the Invention: Medical catheter and medical catheter system) discloses a special catheter comprising an outer tube having a balloon at its distal end and an inner tube inserted into the outer tube, wherein, after insertion to a target position in the bronchus, the balloon is inflated to block the bronchus, a drug is injected through the inner tube, and then only the inner tube can be withdrawn (see Fig. 2). This prior art is a hemostatic balloon catheter that can be used without the aid of a bronchoscope; however, it cannot be used simultaneously with a bronchoscope, making it difficult to cope with urgent situations.

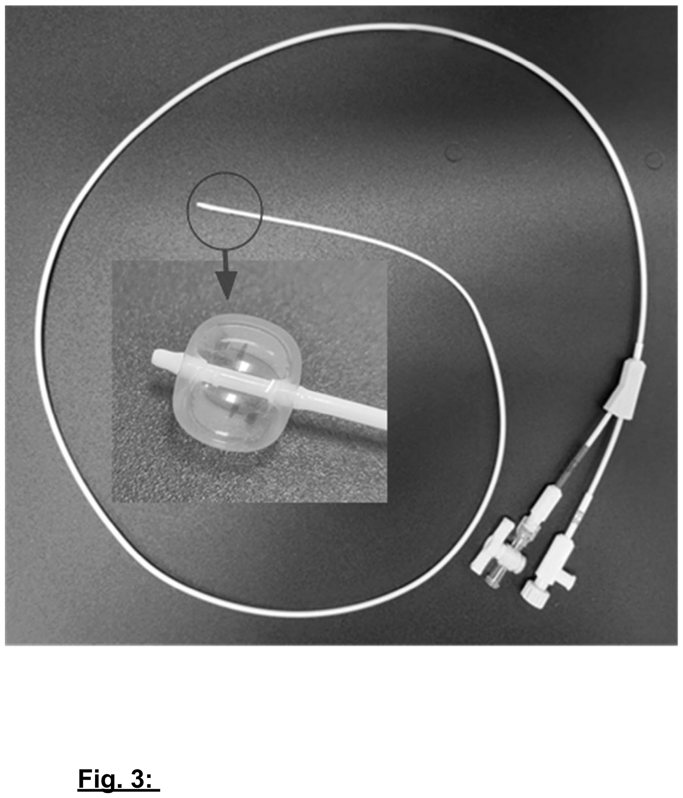

In the medical field, in cases where bleeding is anticipated, a balloon occlusion catheter having a balloon formed at its distal end (see Fig. 3) may be inserted through the working channel of a bronchoscope into an appropriate position at the bronchial orifice connected to the lesion, and the procedure may then be performed in such a state. In this state, even when severe bleeding obstructs the field of view, hemostasis can be achieved by inflating the balloon of the catheter to block the bronchus having the lesion.

However, according to such a method, movement of the bronchoscope occurs during the procedure and may cause the balloon catheter to be displaced from its initially set position or to advance further inside (dislocation), thereby leading to a dangerous situation in which the bronchus is not actually blocked but is mistakenly assumed to be hemostatic. Furthermore, even if the balloon catheter remains in position, when it is inserted into the bronchus connected to the lesion for prophylactic hemostasis and the procedure is performed, the already narrow bronchus is further obstructed by the balloon catheter, making it difficult for the bronchoscope to advance, or even if advanced, the working space of the bronchoscope becomes restricted. In addition, even when the balloon catheter functions at the desired position, because it is inserted through the working channel of the bronchoscope, the bronchoscope must remain inserted in the patient's body until hemostasis is completed and the operation of the balloon catheter is released. Accordingly, not only is there waste in that the bronchoscope cannot be used during this period, but also the prolonged insertion of the bronchoscope into the patient's body exposes the patient to discomfort and risk.

### Detailed Description of the Invention

### Technical Problem

It is an object of the present invention to provide a hemostatic balloon catheter of a novel structure which, during bronchoscopy, is secured in advance independently of the bronchoscope adjacent to the bronchus in which the lesion is located, and which can, upon withdrawal of the bronchoscope from the bronchus, selectively inflate a balloon to occlude the bronchus as required.

### Technical Solution

In order to achieve the above-mentioned object,
the present invention provides a bronchial hemostatic balloon catheter,
characterized in that a securing balloon is provided at a distal end to be inserted into the body, and one or more hemostatic balloons are provided at a predetermined distance from the securing balloon.

### Effects of the Invention

According to the present invention as described above, since the catheter is secured in advance, independently of the bronchoscope, at a location where bleeding is likely to occur and is maintained in a hemostasis-ready state, immediate and precise hemostasis can be achieved by slightly retracting the bronchoscope when bleeding occurs during specimen collection, and the bronchoscope can also be utilized for other appropriate treatments. Accordingly, the invention contributes to patient safety and significantly reduces the burden on medical personnel.

### Brief Description of the Drawings

FIGS. 1 and 2 are exemplary views of hemostatic balloon catheters according to the prior art.
FIG. 3 is an exemplary photograph of a balloon catheter used for bronchial hemostasis in conventional procedure settings.
FIG. 4 illustrates an exemplary conceptual and partial cross-sectional views of a hemostatic balloon catheter according to the present invention.
FIG. 5 is a conceptual diagram illustrating the operational sequence of the bronchial hemostatic balloon catheter according to the present invention.

### [Explanation of Reference Numerals]

| | | | |
|---|---|---|---|
| 10. | Securing balloon | 11. | Securing fluid lumen |
| 12. | Securing fluid supply means | 20. | Hemostatic balloon |
| 21. | Hemostatic fluid lumen | 22. | Hemostatic fluid supply means |

### Best Mode for Carrying Out the Invention

The present invention will now be described in further detail with reference to the accompanying drawings and embodiments. However, such drawings and embodiments are merely illustrative for facilitating the understanding of the technical concept and scope of the present invention, and the technical scope of the present invention is not limited or altered thereby. It will be apparent to those skilled in the art that various modifications and alterations can be made within the scope of the technical concept of the present invention based on these examples.

Since the catheter itself, equipped at the distal end of the tube with a balloon portion that can be manipulated externally (from the proximal end), can be manufactured using conventionally known materials and techniques, a detailed description of the method for manufacturing the hemostatic balloon catheter according to the present invention will be omitted. For convenience of description and understanding, the portion toward the inside of the patient's body shall be referred to as the distal end, and the portion toward the outside of the patient's body shall be referred to as the proximal end.

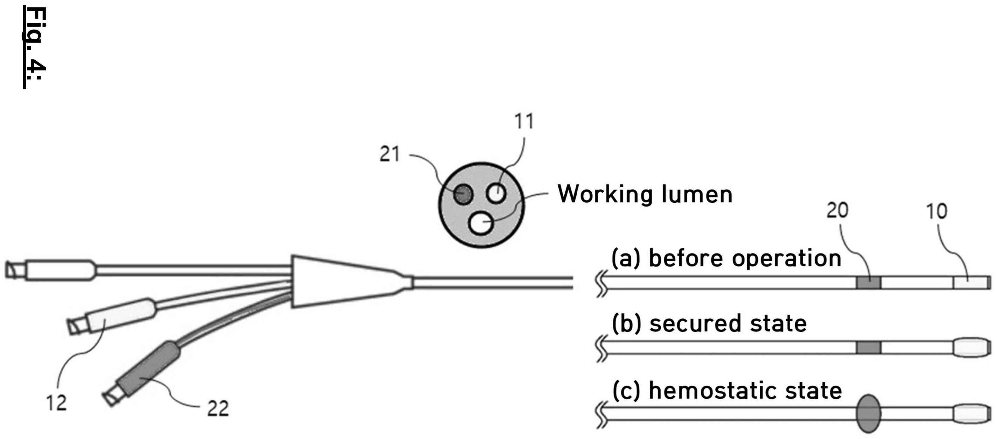

As described above, the present invention relates to a bronchial hemostatic balloon catheter, characterized in that a securing balloon is provided at the distal end to be inserted into the body, and one or more hemostatic balloons are provided at a predetermined distance from the securing balloon. Fig. 4 illustrates exemplary conceptual and partial cross-sectional views of the hemostatic balloon catheter according to the present invention. In the drawings, the balloon portions operated by the same lumen and their corresponding proximal portions are expressed in the same color (shading), which is merely for the purpose of preventing confusion and is not essential. The right side of the drawing illustrates, from top to bottom, the state before use, the state in which only the securing balloon is inflated, and the state in which both the securing balloon and the hemostatic balloon are inflated. While the present invention allows for two or more hemostatic balloons, their function and operation are substantially the same as those of a single hemostatic balloon; therefore, the following description will focus on the case of a single hemostatic balloon as illustrated in the drawings.

As illustrated in Fig. 4, in the present invention, the securing balloon (10) is connected to the securing fluid supply means (12) at the proximal end via the securing fluid lumen (11), and the hemostatic balloon (20) is connected to the hemostatic fluid supply means (22) at the proximal end via the hemostatic fluid lumen (21). Accordingly, the securing balloon and the hemostatic balloon are operated (inflated and deflated) separately or sequentially by independently manipulating the securing fluid supply means and the hemostatic fluid supply means at the proximal end.

Although not illustrated, the present invention may also employ a structure wherein the securing balloon is formed thinner than the hemostatic balloon, and a single fluid lumen and a single fluid supply means are applied. In this case, by controlling the amount of fluid supplied, the securing balloon can be operated (inflated and deflated) first, followed by the hemostatic balloon.

In the present invention, the fluid for inflating and deflating the balloons may be air or physiological saline.

As in the example of Fig. 4, the catheter tube may include an additional working lumen (which is not essential) in addition to the securing fluid lumen and the hemostatic fluid lumen, through which a guidewire may be inserted to control insertion of the catheter into the body, and a predetermined through-hole may be provided at the distal end of the tube, between the securing balloon and the hemostatic balloon, or at a position proximal to the hemostatic balloon, so that necessary drugs can be injected or pulmonary substances can be aspirated and removed.

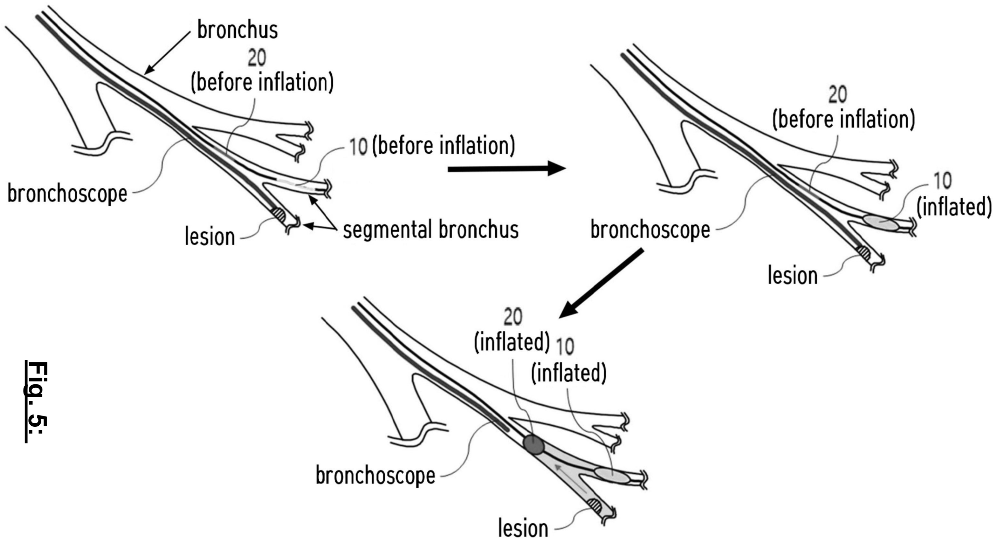

As described above, the operational sequence of the bronchial hemostatic balloon catheter of the present invention will be described with reference to Fig. 5. In the figure, the bronchoscope is schematically represented by a thick line.

First, either using only the hemostatic balloon catheter according to the present invention or using a bronchoscope, the distal end of the hemostatic balloon catheter is introduced into the segmental bronchus adjacent to the site to be treated (e.g., a lesion; hereinafter, "lesion"), and then the securing balloon is appropriately inflated to secure the distal end of the hemostatic balloon catheter. Under this condition, the deflated hemostatic balloon is positioned in the "common upstream bronchus" of the segmental bronchus containing the lesion and the segmental bronchus in which the securing balloon is secured. At this state, necessary procedures, such as collecting a specimen from the lesion, are performed using the bronchoscope. If bleeding occurs during or after the procedure, the bronchoscope is slightly retracted to the proximal side of the hemostatic balloon, and the hemostatic fluid supply means is operated to inflate the hemostatic balloon. The hemostatic balloon blocks the "common upstream bronchus," thereby confining the bleeding to the segmental bronchus containing the lesion.

Thus, according to the present invention, during a procedure using a bronchoscope, the position of the hemostatic balloon is consistently maintained at a predetermined optimal location by the securing balloon, enabling immediate hemostasis at the precise location upon the occurrence of bleeding. Furthermore, according to the present invention, the securing balloon at the distal end of the balloon catheter is positioned and secured in another segmental bronchus, and the hemostatic balloon, in a deflated state, is positioned in the wider "common upstream bronchus"; therefore, unlike the prior art, the balloon catheter is not located in the segmental bronchus containing the lesion. As a result, the bronchoscope can easily enter the segmental bronchus containing the lesion.

Furthermore, through the working lumen exemplified in one embodiment of the present invention, an appropriate drug (e.g., a hemostatic agent) can be administered at an appropriate location, and, if necessary, blood accumulated in the segmental bronchus containing the lesion can be removed without affecting other bronchial regions. In addition, according to the present invention, since the hemostatic balloon catheter operates independently of the bronchoscope, other procedures can be performed with the bronchoscope even during hemostasis, or the bronchoscope can be completely withdrawn from the patient's body, thereby contributing to medical efficiency and patient convenience.

Meanwhile, in the present invention, the securing balloon should not excessively compress the segmental bronchus in which it is secured, yet it must not become dislodged in the inflated state. Therefore, it is preferable that the inflated securing balloon have a structure or be made of a material that prevents dislodgement.

For example, the securing balloon preferably has a cylindrical shape when inflated so that the contact area with the inner surface of the segmental bronchus can be increased. Furthermore, to enable the securing balloon to be more reliably positioned and secured, a plurality of ring-shaped protrusions may be formed on the surface of the securing balloon in a direction perpendicular to the axis of the catheter tube to provide resistance to slipping (not shown).

## Claims

1. A bronchial hemostatic balloon catheter, **characterized in that** a securing balloon is provided at a distal end to be inserted into the body, and one or more hemostatic balloons are provided at a predetermined distance from the securing balloon.

2. A bronchial hemostatic balloon catheter according to Claim 1, wherein the securing balloon and the hemostatic balloon are operated separately or sequentially.

3. A bronchial hemostatic balloon catheter according to Claim 1, wherein the securing balloon is connected to the securing fluid supply means at the proximal end via the securing fluid lumen, and the hemostatic balloon is connected to the hemostatic fluid supply means at the proximal end via the hemostatic fluid lumen.

4. A bronchial hemostatic balloon catheter according to Claim 1, wherein the hemostatic balloon is a single balloon.

5. A bronchial hemostatic balloon catheter according to Claim 1 or Claim 2, wherein the securing balloon has a substantially rectangular shape in longitudinal cross-section when inflated.

6. A bronchial hemostatic balloon catheter according to Claim 1 or Claim 2, wherein the surface of the securing balloon has a structure or is made of a material having high frictional resistance.

7. A bronchial hemostatic balloon catheter according to Claim 6, wherein a plurality of ring-shaped protrusions are formed on the surface of the securing balloon in a direction perpendicular to the axis of the catheter.
